# EUROPÄISCHE PATENTSCHRIFT

(11) **EP 2 480 181 B1**
(45) Veröffentlichungstag und Bekanntmachung des Hinweises auf die Patenterteilung: **23.05.2018**
(21) Anmeldenummer: 10751936.5
(22) Anmeldetag: 08.09.2010
(51) Int. Cl.: A61F 5/058

(54) **FIXIERELEMENT SOWIE ANWENDUNG EINES SOLCHEN FIXIERELEMENTS ZUM FIXIEREN EINES GEGENSTANDES, INSBESONDERE VON GLIEDMASSEN**
FIXING ELEMENT AND USE OF SUCH AN ELEMENT FOR FIXATION OF OBJECTS, IN PARTICULAR LIMBS
ÉLÉMENT DE FIXATION ET UTILISATION D'UN TEL ÉLÉMENT DE FIXATION POUR LA FIXATION D'UN OBJET, NOTAMMENT DE MEMBRES

(30) Priorität: 22.09.2009 CH 14592009; 12.02.2010 CH 1772010
(43) Veröffentlichungstag der Anmeldung: 01.08.2012
(73) Patentinhaber: Chrisofix AG, 8212 Neuhausen am Rheinfall (CH)
(72) Erfinder: BOLLA, Kalman, 8212 Neuhausen am Rheinfall (CH)
(74) Vertreter: Erdély, Péter
(86) Internationale Anmeldenummer: PCT/EP2010/063144
(87) Internationale Veröffentlichungsnummer: WO 2011/036054

(56) Entgegenhaltungen:
- WO-A1-98/49979
- WO-A1-2004/078079
- WO-A1-2007/028199
- CH-A5- 689 820
- US-A- 3 850 167

## Beschreibung

Die vorliegende Erfindung bezieht sich allgemein auf das Gebiet der Befestigungstechnik sowie speziell auf das Gebiet der medizinischen Schienen. Sie betrifft ein Fixierelement gemäss dem Oberbegriff des Anspruchs 1, wie zum Beispiel aus der US 3,850,167 A1 bekannt, sowie verschiedene Anwendungen eines solchen Fixierelements.
Aus der CH 689 820 A5 und der WO-A1 97/22312 ist eine medizinische Schiene bekannt, deren Besonderheit darin besteht, dass sie als Kernelement ein in sich steifes, mit der Hand ohne weitere Hilfsmittel plastisch verformbares Metallblech umfasst, welches gleichmässig gewellt ausgebildet ist, wobei die Berge und Täler der Wellen im Wesentlichen quer zu einer ausgezeichneten Richtung der Schiene, nämlich der Schienenachse, verlaufen. Das gewellte Kernblech, das vorzugsweise aus Aluminium besteht und eine bestimmte Blechdicke von z.B. 0,2 bis 0,4 mm aufweist, ist beidseitig mit einer Abdeckschicht abgedeckt, die an die jeweilige Anwendung angepasst ist. Die innere Abdeckschicht kann nach Art einer Polsterung ausgebildet sein, um den Tragekomfort der Schiene zu verbessern. Die äussere Abdeckschicht kann abwaschbar ausgebildet sein, um die Schiene unempfindlicher gegen äussere Einflüsse zu machen.
Der Kerngedanke bei dieser Art von Schiene ist, dass durch die wellige Ausbildung des Kernblechs eine "Flächenreserve" bereit steht, welche die Anpassung der Schiene an unregelmässige Körperformen erleichtert, indem beim Verformen mit der Hand Wellen lokal flach gedrückt oder aufgesteilt werden können, um eine lokale flächige Ausdehnung oder Kontraktion der Schiene zu ermöglichen. Auf diese Weise wird ein Knittern oder Knicken des Bleches - wie es bei der Verformung eines ebenen Bleches zu erwarten wäre - sicher vermieden. Darüber hinaus ergibt sich aufgrund der Erstreckung der Wellen des Kernblechs quer zur Schienenrichtung beim Anformen der Schiene an den zu schienenden Körperteil eine Versteifung der Schiene insgesamt, so dass sich eine Schiene mit nahezu idealen Eigenschaften ergibt, die schnell und ohne Hilfsmittel an den zu schienenden Körperteil angepasst werden kann, leicht und komfortabel zu tragen ist, und beim Anformen die erwünschte hohe Steifigkeit erhält.

Zur Befestigung derartiger Schienen wurden bisher als Fixierelemente elastische bzw. flexible, mehrheitlich textilartige, Bänder verwendet, die selber die Bewegung des geschienten Körperteils nicht einschränken. Medizinische Schienen fixiert mit solchen Elementen sind bekannt unter anderen von WO 98/49979 und WO 2007/128 199 A1. In manchen Fällen ist es jedoch wünschenswert, Fixierelemente zu haben, mit denen der Schienungseffekt verstärkt werden kann, und die auch für andere Anwendungen geeignet sein sollen.

Es ist daher Aufgabe der Erfindung, ein Fixierelement zu schaffen, das besonderes für medizinische Schienen, aber darüber hinaus auch für andere Anwendungen wie z.B. das Verpacken von Gegenständen geeignet sind und auf einfache Weise ohne zusätzliche Hilfsmittel eine genaue und sichere Fixierung ermöglichen, sowie Anwendungen eines solchen Fixierelements anzugeben.

Die Aufgabe wird durch die Ansprüche 1, 6 und 9 gelöst.

Charakteristisch ist bei dem erfindungsgemässen Fixierelement, dass die Wellen des gewellten Kernblechs im Wesentlichen parallel zur Vorzugsrichtung orientiert sind, und dass am Fixierelement Mittel zum lösbaren Befestigen des Fixierelements angeordnet sind. Durch die Orientierung der Wellen parallel zur Vorzugsrichtung kann das Fixierelement in der Vorzugsrichtung Zug- und Druckkräfte aufnehmen, ohne seine Länge zu ändern. Gleichzeitig ist durch die Welligkeit des Kernblechs eine Verformbarkeit gegeben, die eine gute Anpassbarkeit des Fixierelements an unregelmässige Formen mittels der Hand ergibt.

Das Kernblech besteht vorzugsweise aus Aluminium und weist eine Dicke zwischen 0,2 mm und 1,2 mm auf. Damit ist es beständig gegen Umwelteinflüsse und lässt sich je nach Einsatzzweck mehr oder weniger gut verformen.

Das Fixierelement kann ohne zusätzliche Hilfsmittel einfach und wiederholbar und mit hoher Haftkraft befestigt werden, wenn gemäss einer Ausgestaltung der Erfindung die Befestigungsmittel nach Art eines Klettverschlusses ausgebildet sind.

Zum Schutz des Kernblechs und der zu fixierenden Gegenstandes sowie zur weiteren Verbesserung der mechanischen Eigenschaften kann das Kernblech ein- oder beidseitig mit einer flexiblen Abdeckschicht, vorzugsweise aus einem Schaumstoff oder Gewebe, abgedeckt sein.
Eine erfindungsgemässe Anwendung des Fixierelements besteht im Fixieren eines Gegenstandes auf bzw. in einer stabilen Unterlage, wobei der Gegenstand auf bzw. in die Unterlage gelegt und mittels eines oder mehrerer Fixierelemente, welche über den Gegenstand geführt und an der Unterlage befestigt werden, gegen eine Bewegung relativ zur Unterlage gesichert wird.

Dabei werden das bzw. die Fixierelement(e) lösbar an der Unterlage befestigt. Insbesondere werden zur Anpassung des Fixierelements an eine Vertiefung oder Erhöhung in der Aussenkontur des Gegenstandes die Wellen des Kernblechs lokal mit der Hand flach gedrückt.

Der Gegenstand kann mit Vorteil eine Gliedmasse sein, wobei die Unterlage Teil einer medizinischen Schiene ist, und die Gliedmasse in der Schiene fixiert wird.

In besonderen Fällen können an der medizinischen Schiene zusätzliche Fixierelemente der erfindungsgemässen Art angebracht sein, welche die Schiene in einer bestimmten, insbesondere abgewinkelten Konfiguration stabilisieren.

Die Erfindung soll nachfolgend anhand von Ausführungsbeispielen im Zusammenhang mit der Zeichnung näher erläutert werden. Es zeigen
- Fig. 1: In einer perspektivischen Darstellung die Fixierung eines Gegenstandes auf einer Unterlage gemäss einem Ausführungsbeispiel der Erfindung;
- Fig. 2: die Draufsicht auf (Fig. 2a) und der Längsschnitt durch (Fig. 2b) ein beispielhaftes, lösbar zu befestigendes Fixierelement für eine Vorrichtung nach Fig. 1;
- Fig. 3: eine Ellbogenschiene bekannter Art mit flexiblen Befestigungsbändern;
- Fig. 4: in einer zu Fig. 2b vergleichbaren Darstellung ein Fixierelement nach der Erfindung mit abweichenden Befestigungsmitteln;
- Fig. 5: eine Ellbogenschiene gemäss einem Ausführungsbeispiel der Erfindung mit in sich steifen, mit der Hand plastisch verformbaren, streifenförmigen Fixierelementen sowie einem zusätzlichen seitlichen Fixierelement im Biegungsbereich;
- Fig. 6: die Ellbogenschiene aus Fig. 5 mit einem zusätzlichen unteren Fixierelement im Biegungsbereich;
- Fig. 7: in der Draufsicht von oben ein weiteres Ausführungsbeispiel für ein Fixierelement nach der Erfindung, welches ganzflächig mit lösbaren Befestigungsmitteln nach Art eines Klettverschlusses ausgerüstet ist;und
- Fig. 8: den Querschnitt durch das Fixierelement nach Fig. 7, wobei die klettverschlussartigen Abdeckschichten noch einmal separat vergrössert dargestellt sind.

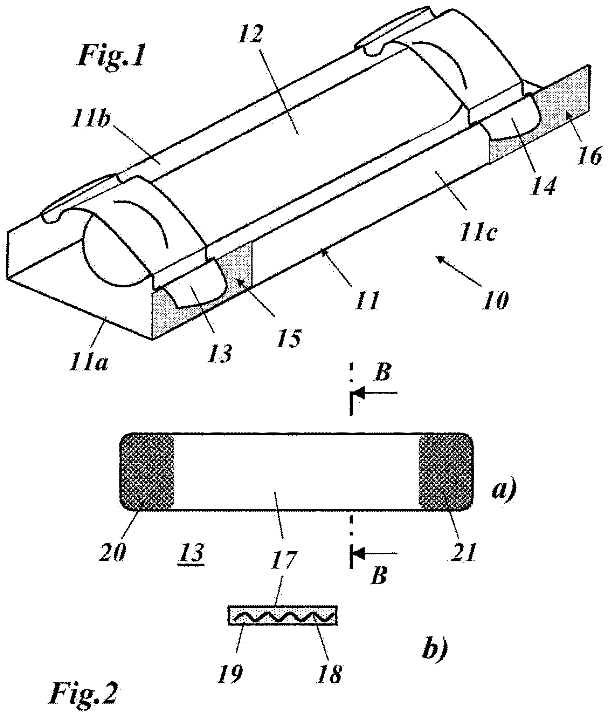

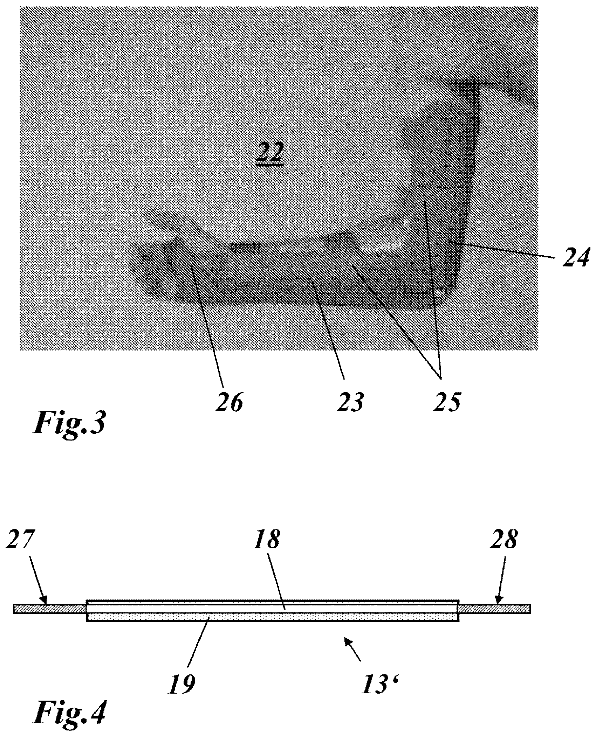

Fig. 2, 4, 7 und 8 zeigen Ausführungsbeispiele für ein Fixierelement nach der Erfindung. Die Fixierelemente 13 und 13' der Figs. 2 und 4 werden nachfolgend im Zusammenhang mit dem Einsatz bei einer Fixiervorrichtung 10 (Fig. 1) und einer Ellbogenschiene 22 (Fig. 3) erläutert. Es versteht sich von selbst, dass im Rahmen der Erfindung andere Einsatzmöglichkeiten denkbar und vorteilhaft sind.

Fig. 3 zeigt eine Ellbogenschiene bekannter Art. Die Ellbogenschiene 22 umfasst ein Unterarmteil 23 und ein Oberarmteil 24, die zueinander nahezu einen rechten Winkel bilden. Beide Teile 23, 24 haben eine näherungsweise U-förmige Querschnittskontur, die den Unterarm bzw. den Oberarm soweit aufnimmt, dass er nach oben nur geringfügig übersteht. Zur Befestigung der Ellbogenschiene 22 am Arm sind im Stand der Technik mehrere flexible Befestigungsbänder 25 vorgesehen, die über den Arm laufend von einer Seite der Schiene zur anderen Seite reichen und dort lösbar befestigt sind. Die Ellbogenschiene 22 kann auf der Innenseite zur Erhöhung des Tragekomforts eine zusätzliche Polsterung aufweisen und perforiert sein, um Luft an den in der Schiene liegenden Arm zu lassen.

Ein kritischer Bereich der bekannten Ellbogenschiene 22 ist der vordere Endbereich, in dem die Hand zu liegen kommt. Die Hand ist weitgehend hochkant in der Schiene positioniert und soll durch ein längeres Befestigungsband 26, das zwischen Daumen und Zeigefinger hindurchgeführt wird, in der Schiene fixiert werden. Da auch dieses Befestigungsband 26 flexibel ausgebildet ist, ist die Fixierung in vielen Fällen ungenügend: Die Hand kann trotz des Befestigungsbandes 26 beispielsweise um ihre Längsachse gedreht werden, was in bestimmten Verletzungssituationen des Armes problematisch ist und die schienende Wirkung mehr oder weniger stark vermindert.

Um hier Abhilfe zu schaffen, schlägt die Erfindung vor, die flexiblen Befestigungsbänder 26 und ggf. auch 25 durch spezielle, insbesondere streifenförmige, Fixierelemente zu ersetzen, wie sie beispielhaft in Fig. 2 und Fig. 4 gezeigt sind.

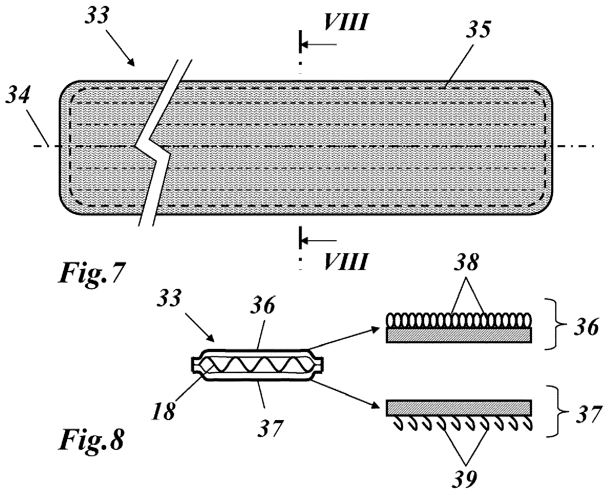

Das Fixierelement 13 der Fig. 2 zeichnet sich durch seine speziellen Verformungs- und Stabilitätseigenschaften aus. Es basiert auf einem gewellten Kernblech 18, vorzugsweise aus Aluminium, wie es bereits früher zum Einsatz als Schienenmaterial vorgeschlagen worden ist (siehe die WO-A1-97/22312). Die dortige Offenbarung ist Teil der vorliegenden Anmeldung und gibt Auskunft über die bevorzugten Abmessungen und sonstige Eigenschaften des gewellten Kernblechs 18. Anders als bei dem Schienenmaterial gemäss der WO-A1-97/22312 erstrecken sich die Wellen nicht mehr quer, sondern nunmehr parallel zur Längsrichtung (entspricht der Vorzugsrichtung 34 in Fig. 7). Diese Orientierung des Kernblechs gibt dem Fixierelement 13 besondere Eigenschaften. Bei geeigneter Blechdicke kann das Kernblech 18 ohne weitere Hilfsmittel mit der Hand verformt werden, wobei die Wellen durch lokales Stauchen steiler werden und grössere Wellenhöhen ausbilden bzw. durch lokales Dehnen flacher werden oder ganz verschwinden. Auf diese Weise kann das gewellte Kernblech einer unebenen Aussenkontur angepasst bzw. angeformt werden, ohne dass seine Steifigkeit verloren geht. Im Gegenteil: Durch die Verformung der Wellen erhöht sich die Steifigkeit des Materials, so dass es noch besser eine stützende Funktion ausüben kann. Gleichzeitig stellt die Ausrichtung der einzelnen Wellen parallel zur Längsrichtung bzw. Vorzugsrichtung 34 sicher, dass in dieser Richtung eine Streckung oder Stauchung des Materials mit entsprechender Längenänderung weitgehend ausgeschlossen ist. Das Kernblech (18) ist mit einer Abdeckung (17, 19) versehen.

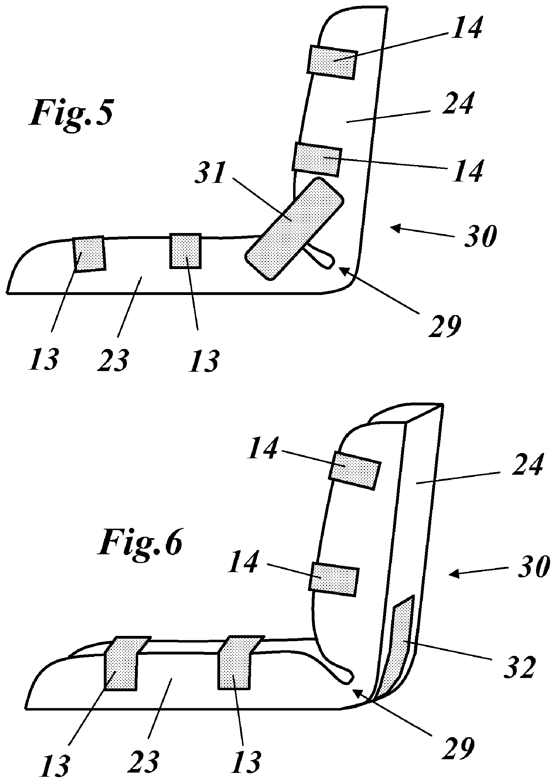

Wird nun ein solches streifenförmiges oder auch grösserflächiges und ggf. unregelmässig geformtes Fixierelement 13 anstelle des flexiblen Befestigungsbandes 26 bei einer Ellbogenschiene 22 gemäss Fig. 3 (siehe die Ellbogenschiene in Fig. 5 oder 6) oder einer anderen Schiene, wie zum Beispiel der eingangs beschriebenen, eingesetzt, kann das Fixierelement an die in der gezeigten Position befindliche Hand angeformt werden und hält aufgrund seiner Steifigkeit die Hand sicher in dieser Position. Ein unbeabsichtigtes, schädliches Verdrehen der Hand ist dann nicht mehr möglich.

Wird ein solches Fixierelement 13 - wie in Fig. 3 gezeigt - bei einer medizinischen Schiene eingesetzt, ist es vorteilhaft, wenn es ein- oder beidseitig mit einer Abdeckung 19 versehen ist, die den direkten Kontakt der Haut mit dem Kernblech 18 verhindert und beispielsweise aus einem Schaumstoff oder einem Gewebe besteht. Darüber hinaus kann das Fixierelement perforiert sein, um einen Luftaustausch mit abgedeckten Körperpartien zu ermöglichen.

Zur lösbaren Befestigung des Fixierelements 13 an der Schiene kann das Fixierelement 13 an den Enden mit oberflächlich aufgebrachten Befestigungsmitteln 20 und 21 versehen sein, die als mit Haken versehene Bereiche (siehe auch Fig. 8) Teil eines Befestigungssystems nach Art eines Klettverschlusses sind. Ein mit entsprechenden Ösen versehener Velours kann als Gegenstück auf der Oberfläche der Schiene angeordnet sein. Besonders flexibel ist das System einsetzbar, wenn die gesamte Aussenfläche der Schiene mit einer als Teil eines Klettverschlusses dienenden Abdeckschicht, z.B. mit einem Schlingenvelours, ausgerüstet ist, so dass die Fixierelemente an beliebigen Stellen der Aussenseite befestigt werden können.

Anstelle eines integrierten Klettverschlusssystems kann gemäss Fig. 4 ein Fixierelement 13' aber auch mit Befestigungsmitteln 27, 28 ausgestattet sein, die als flexible, vorzugsweise nicht dehnbare, Laschen an den Enden des Elements angeordnet sind und Haken, Druckknöpfe oder andere Mittel zum lösbaren Befestigen aufweisen. Es ist aber auch denkbar, die Fixierelemente lösbar (oder nicht lösbar) an der Schiene anzukleben. Ein einseitiges Anformen der Fixierelemente an die Schiene in einer einstückigen Ausgestaltung bringt dagegen den Nachteil, dass beim Zuschneiden des Grundmaterials vergleichsweise viel Material verloren geht.

Es ist unmittelbar einleuchtend, dass im Rahmen der Erfindung Fixierelemente 13, 13' der in Fig. 2 und Fig. 4 gezeigten Art nicht nur bei einer Ellbogenschiene gemäss Fig. 3 Anwendung finden können, sondern auch bei anderen medizinischen Schienen, wo eine stabile und genaue Fixierung eines geschienten Teils relativ zur Unterlage einfach und sicher erreicht werden muss. Hierzu sei insbesondere auch auf Oberarmschienen verwiesen, bei denen die erfindungsgemässe Kombination aus schienenartiger Unterlage und Fixierelementen dazu beiträgt, die Schiene am Oberarm bei gleichzeitig hohem Tragekomfort so an die jeweilige Form des Oberarms anzupassen und zu fixieren, dass die Schiene nicht verrutschen kann und dennoch ausreichend Bewegungsspielraum gewährt.

Beim Einsatz der erfindungsgemässen Fixierelemente in medizinischen Schienen kann durch Variation der Anzahl, der Elementegeometrie und der Blechdicke, ausgehend von einer U-förmigen Basisschiene der gesamte Bereich von einer semi-zirkulären Schienung bis zu einer vollen zirkulären Schienung nahtlos überdeckt werden, wobei durch die speziellen Eigenschaften des Schienenmaterials in jedem Fall eine hohe Anpassbarkeit und ein hoher Tragekomfort gewährleistet sind.

Darüber hinaus können - wie dies anhand der Ellbogenschiene 30 aus Fig. 5 und 6 gezeigt ist - zusätzliche Fixierelemente 31, 32 eingesetzt werden, um die Ellbogenschiene (oder andere Schienen) in einer bestimmten (winkligen) Konfiguration zusätzlich zu stabilisieren. Die Fixierelemente 31, 32 sind analog aufgebaut wie die Fixierelemente 13, 13' und 14 und weisen daher eine entsprechende Eigensteifigkeit und Zugfestigkeit in Längsrichtung auf. Wenn die Ellbogenschiene 30 der Fig. 5 in einem Biegungsbereich 29 annähernd rechtwinklig oder mit einem anderen vorbestimmten Winkel abgebogen worden ist, kann diese Winkelstellung auf einfache Weise dadurch stabilisiert werden, dass das eigensteife

Fixierelement 31 nach Art einer Verstrebung seitlich schräg über den Winkelbereich verlaufend befestigt wird, um eine Winkeländerung im Biegungsbereich 29 sicher zu unterbinden.

Solche Fixierelemente 31 können sowohl nur auf einer Seite als auch auf beiden Seiten der Schiene angebracht werden.

Es ist aber auch denkbar, die Ellbogenschiene 30 oder vergleichbare Schienen gemäss Fig. 6 auf andere Weise durch zusätzliche, insbesondere aussen aufgebrachte Fixierelemente 32 zu stabilisieren bzw. zu versteifen, wenn in bestimmten Situationen die Eigensteifigkeit des Schienenmaterials nicht ausreicht. Eine solche Versteifung kann - wie in Fig. 6 gezeigt - in einem Biegungsbereich 29 angebracht sein. Sie kann aber auch an anderen stärker belasteten Stellen der Schiene vorgesehen werden. In diesem Fall ist es vorteilhaft, grössere Blechdicken im Bereich von 0,6 mm bis 1,2 mm zu nehmen.

Die Erfindung kann aber weit über den Bereich der medizinischen Schienen hinaus ganz allgemein dort eingesetzt werden, wo ein Gegenstand mit einer bestimmten Aussenkontur auf einer stabilen Unterlage schnell und sicher fixiert werden muss. Ein solcher allgemeiner Anwendungsfall ist in Fig. 1 illustriert. Ein - in diesem Fall zylindrischer - Gegenstand oder Körper 12, der auf einer stabilen Unterlage 11 mit Grundplatte 11a und parallelen Seitenwänden 11b, 11c liegt, soll auf dieser Unterlage 11 so fixiert werden, dass er sich relativ zur Unterlage 11 nicht bewegen (wegrollen oder verschieben) kann. Die Enden des Gegenstandes 12 werden dazu mit zwei streifenförmigen Fixierelementen 13 und 14 der in Fig. 2 oder 4 gezeigten Art fixiert, indem die Elemente an die kreisförmigen Kanten an den Enden des Zylinders und an die Seitenwände 11b, 11c der Unterlage 11 angeformt werden. Zur Befestigung der angeformten Fixierelemente 13 und 14 sind auf den Aussenseiten der Seitenwände 11b, 11c beispielsweise Befestigungsbereiche 15 und 16 vorgesehen, die fein verteilt kleine Schlingen aufweisen, in welche das Fixierelement 13 aus Fig. 2 mit seinen Befestigungsmitteln 20, 21 nach Art eines Klettverschlusses flächig einhaken kann. Andere Arten der lösbaren oder nicht lösbaren Befestigung sind aber auch denkbar.

Durch die inhärente Steifigkeit der Fixierelemente 13, 13' mit dem gewellten Kernblech 18 können auch schwerere Körper auf der Unterlage 11 gegen Verschieben oder Wegrollen gesichert werden, wobei die Anformung an die Aussenkontur ohne Hilfsmittel nur mit der Hand schnell und einfach durchgeführt werden kann.

Besonders vielfältig sind die Einsatzmöglichkeiten der erfindungsgemässen Fixierelemente, wenn sie, wie das Fixierelement 33 der Fig. 7 und 8, auf den gegenüberliegenden Seiten ganzflächig mit Abdeckschichten 36 und 37 versehen sind, die als Gegenstücke gemeinsam einen Klettverschluss bilden können, indem auf Oberfläche der einen Abdeckschicht 36 Schlingen 38 ausgebildet sind, während auf der Oberfläche der anderen Abdeckschicht entsprechende Haken 39 angeordnet sind. Die Abdeckschichten 36 und 37 sind randseitig beispielsweise durch eine Randnaht 35 miteinander verbunden und bilden so eine geschlossene Tasche, in der sich das gewellte Kernblech 18 befindet, dessen Wellen - wie in Fig. 7 durch die gestrichelten Linien angedeutet - parallel zur Vorzugsrichtung 34 ausgerichtet sind.

### Bezugszeichenliste

- 10: Fixiervorrichtung
- 11: Unterlage (stabil)
- 11a: Grundplatte
- 11b,c: Seitenwand
- 12: Gegenstand
- 13, 13', 14: Fixierelement (z.B. streifenförmig)
- 15, 16: Befestigungsbereich
- 17: Streifen
- 18: Kernblech (gewellt)
- 19: Abdeckung
- 20, 21: Befestigungsmittel (z.B. klettbandartig)
- 22, 30: Ellbogenschiene
- 23: Unterarmteil
- 24: Oberarmteil
- 25, 26: Befestigungsband
- 27, 28: Befestigungsmittel
- 29: Biegungsbereich
- 31, 32, 33: Fixierelement
- 34: Vorzugsrichtung
- 35: Randnaht
- 36, 37: Abdeckschicht
- 38: Schlinge
- 39: Haken

## Patentansprüche

1. Fixierelement (13, 13', 14, 31, 32, 33), welches als ein flächiges, insbesondere streifenförmiges Element mit zwei in Längsrichtung gegenüberliegenden Endabschnitten ausgebildet ist, und dieses Fixierelement (13, 13', 14, 31, 32, 33) eine Vorzugsrichtung (34) aufweist, entlang welcher das Fixierelement an einer bzw. um eine Unterlage (11) zu spannen ist, wobei die Unterlage (11) zum Halten eines auf oder in der Unterlage (11) angeordneten Gegenstandes (12) geeignet ist, **dadurch gekennzeichnet, dass** das Fixierelement
- ein Kernblech (18) mit einer äußeren Abdeckschicht (17, 19, 36, 37) an seinen beiden Seiten, und
- wenigstens an seinen in Längsrichtung gegenüberliegenden Endabschnitten Mittel (20, 21, 27, 28, 38, 39) zum lösbaren Befestigen des Fixierelementes (13, 13', 14, 31, 32, 33) an der bzw. um die Unterlage (11) umfasst, und wobei
- das Kernblech (18) ein in sich steifes, aber manuell verformbares, gewelltes Metallblech ist, und
- die Wellen des metallischen Kernblechs (18) im Wesentlichen parallel zur Vorzugsrichtung (34) orientiert sind.

2. Fixierelement nach Anspruch 1, **dadurch gekennzeichnet, dass** das gewellte Kernblech (18) aus Aluminium besteht.

3. Fixierelement nach Anspruch 1 oder 2, **dadurch gekennzeichnet, dass** das gewellte Kernblech (18) eine Dicke zwischen 0,2 mm und 1,2 mm aufweist.

4. Fixierelement nach einem der Ansprüche 1 bis 3, **dadurch gekennzeichnet, dass** die Befestigungsmittel (20, 21, 27, 28, 38, 39) nach Art eines Klettverschlusses ausgebildet sind.

5. Fixierelement nach einem der Ansprüche 1 bis 4, **dadurch gekennzeichnet, dass** die äußeren Abdeckschichten (17, 19, 36, 37) aus einem flexiblen Material bestehen, das aus einer Gruppe aus Schaumstoff und Gewebe ausgewählt ist.

6. Anwendung eines Fixierelements nach einem der Ansprüche 1 bis 5 zum Fixieren eines Gegenstandes (12) auf bzw. in einer stabilen Unterlage (11), wobei der Gegenstand (12) auf bzw. in die Unterlage (11, 23, 24, 30) gelegt und mittels eines oder mehrerer Fixierelemente (13, 13', 14, 31, 32, 33), welche über den Gegenstand (12) geführt und an der Unterlage (11) befestigt werden, gegen eine Bewegung relativ zur Unterlage (11) gesichert wird.

7. Anwendung nach Anspruch 6, **dadurch gekennzeichnet, dass** das bzw. die Fixierelement(e) (13, 13', 14, 31, 32, 33) lösbar an der Unterlage (11) befestigt werden.

8. Anwendung nach Anspruch 6 oder 7, **dadurch gekennzeichnet, dass** zur Anpassung des Fixierelements (13, 14; 13', 31, 32, 33) an eine Vertiefung oder Erhöhung in der Aussenkontur des Gegenstandes (12) die Wellen des Kernblechs (18) lokal mit der Hand flach gedrückt werden.

9. Vorrichtung zum Fixieren eines Gegenstandes (12), welche Vorrichtung eine stabile Unterlage (11, 23, 24, 30) zum Halten eines Gegenstandes (12) und wenigstens ein Fixierelement (13, 13', 14, 31, 32, 33) nach einem der Ansprüche 1 bis 5 enthält, **dadurch gekennzeichnet, dass**
die Unterlage (11, 23, 24, 30) ein Befestigungsmittel-Gegenstück zum Verbinden mit den Befestigungsmitteln des wenigstens eines Fixierelementes (13, 13', 14, 31, 32, 33) aufweist.

10. Vorrichtung nach Anspruch 9, **dadurch gekennzeichnet, dass** die Unterlage eine medizinische Schiene (22, 30) ist.

## Claims

1. A fastening element (13, 13', 14, 31, 32, 33) formed as a flat, in particular strap-like element with two longitudinally opposite end portions, said fastening element (13, 13', 14, 31, 32, 33) having a preferred direction (34), along which the fastening element is to be tensioned on or around a support (11), wherein the support (11) is adapted for immobilizing an object (12) arranged on or in the support (11), **characterized in that** the fastening element comprises
- a core plate (18) with an external covering layer (17, 19, 36, 37) on both sides thereof, and
- at least on its longitudinally opposite end portions, means (20, 21, 27, 28, 38, 39) for releasably attaching the fastening element (13, 13', 14, 31, 32, 33) on or around said support (11),
and wherein
- the core plate (18) is a rigid but manually deformable, corrugated metal plate, and
- the corrugations of the metal core plate (18) are oriented substantially in parallel to the preferred direction (34).

2. The fastening element of claim 1, **characterized in that** the corrugated core plate (18) is made of aluminium.

3. The fastening element of claim 1 or 2, **characterized in that** the corrugated core plate (18) has a thickness between 0,2 mm and 1,2 mm.

4. The fastening element of any one of claims 1 to 3, **characterized in that** said attaching means (20, 21, 27, 28, 38, 39) are hook and loop fasteners.

5. The fastening element of any one of claims 1 to 4, **characterized in that** the external covering layers (17, 19, 36, 37) are made of a flexible material selected from the group of fabric and foam.

6. Use of a fastening element according to any of claims 1 to 5 for fixing an object (12) on or in a stable support (11), wherein the object (12) is put onto or into the support (11, 23, 24, 30) and immobilized with respect to the support (11) by means of one or more fastening elements (13, 13', 14, 31, 32, 33) that are lead over the object (12) and attached to the support (11).

7. The use of claim 6, **characterized in that** the fastening element(s) (13, 13', 14, 31, 32, 33) is releasably attached to the support (11).

8. The use of claim 6 or 7, **characterized in that** for fitting the fastening element (13, 13', 14, 31, 32, 33) to a recess or a projection of the contour of the object (12), the corrugations of the core plate (18) are locally pressed by hand so as to make it flat.

9. An apparatus for fastening an object (12), said apparatus comprising a stable support (11, 23, 24, 30) for holding an object (12) and at least one fastening element (13, 13', 14, 31, 32, 33) according to any one of claims 1 to 5, **characterized in that** the support (11, 23, 24, 30) comprises counterpart attaching means for coupling with the attaching means of the at least one fastening element (13, 13', 14, 31, 32, 33).

10. The apparatus of claim 9, **characterized in that** the support (11) is a medical splint (22, 30).

## Revendications

1. Élément de fixation (13, 13', 14, 31, 32, 33), étant formé comme un élément plat, en particulier en forme d'une lame avec deux sections finales opposées dans le sens longitudinal, cet élément de fixation (13, 13', 14, 31, 32, 33) ayant un sens préféré (34), selon duquel l'élément de fixation est à tendre sur ou autour d'une semelle (11), la dite semelle (11) étant conformée pour tenir un objet (12) arrangé sur ou dans la semelle (11),
**caractérisé en ce que**
l'élément de fixation comprend
- une tôle de coeur (18) avec une couche recouvrante (17, 19, 36, 37) extérieure sur ses deux côtés, et
- au moins sur ses sections finales opposées dans le sens longitudinal des moyens (20, 21, 27, 27, 28, 38, 39) pour une fixation détachable de l'élément de fixation (13, 13', 14, 31, 32, 33) sur ou autour de la semelle (11),
et
- la tôle de coeur (18) est une tôle métallique ondulée, rigide en soi, mais pouvant être déformée manuellement, et
- les ondes de la tôle de coeur (18) métallique sont orientées essentiellement parallèlement au sens préféré (34).

2. Élément de fixation selon la revendication 1, **caractérisé en ce que** la tôle de coeur (18) ondulée est faite de l'aluminium.

3. Élément de fixation selon la revendication 1 ou 2, **caractérisé en ce que** la tôle de coeur (18) ondulée a une épaisseur entre 0,2 mm et 1,2 mm.

4. Élément de fixation selon l'une des revendications 1 à 3, **caractérisé en ce que** les moyens de fixation (20, 21, 27, 27, 28, 38, 39) sont faites en forme d'une fermeture de type velcro.

5. Élément de fixation selon l'une des revendications 1 á 4, **caractérisé en ce que** les couches recouvrantes (17, 19, 36, 37) extérieures sont faites d'un matériau flexible, choisi d'une groupe comprenant un matériau mousseux et un tissu.

6. Utilisation d'un élément de fixation selon l'une des revendications 1 á 5 pour la fixation d'un objet (12) sur ou dans une semelle (11) stable, où l'objet (12) est mis sur ou dans la semelle (11, 23, 24, 30) et il est assuré à l'aide d'un ou plusieurs éléments de fixation (13, 13', 14, 31, 32, 33) guidés à travers l'objet (12) et fixés sur la semelle (11), contre un mouvement relatif à la semelle (11).

7. Utilisation selon la revendication 6, **caractérisé en ce que** les éléments de fixation (13, 13', 14, 31, 32, 33) sont fixés détachablement sur la semelle (11).

8. Utilisation selon la revendication 6 ou 7, **caractérisé en ce que** pour l'adaptation des éléments de fixation (13, 13', 14, 31, 32, 33) à un creusement ou une élévation dans le contour extérieur des objets (12), les ondes de la tôle de coeur (18) sont comprimés localement manuellement à plat.

9. Dispositif d'immobilisation pour fixer un objet (12), le dispositif comprenant une semelle (11, 23, 24, 30) stable pour supporter l'objet (12) et au moins un élément de fixation (13, 13', 14, 31, 32, 33) selon l'une des revendications 1 á 5, **caractérisé en ce que** la semelle (11, 23, 24, 30) comporte une contre-pièce aux moyens de fixation pour joindre l'au moins un élément de fixation (13, 13', 14, 31, 32, 33) aux moyens de fixation.

10. Dispositif selon la revendication 9, **caractérisé en ce que** la semelle est une éclisse médicale (22, 30).
